# DEMANDE DE BREVET EUROPEEN

(11) **EP 2 072 078 A1**
(43) Date de publication de la demande: **24.06.2009**
(21) Numéro de dépôt: 07405362.0
(22) Date de dépôt: 20.12.2007
(51) Int. Cl.: A61M 39/10, F16L 37/02

(54) **Elément d'assemblage étanche et dispositif utilisant cet élément**

(71) Demandeur: Rochat, Jean-Denis, 1272 Genolier (CH)
(72) Inventeur: Rochat, Jean-Denis, 1272 Genolier (CH)
(74) Mandataire: Savoye, Jean-Paul

(57) **Abrégé**

Cet élément d'assemblage conique étanche en matière plastique pour dispositif d'assemblage mâle-femelle de type Luer Lock, comprend un cône mâle (2, 12) entouré d'une jupe tubulaire (3, 13) concentrique ménageant un espace annulaire autour du cône mâle pour recevoir un élément d'assemblage à cône femelle (5, 15) et dont la paroi interne (4, 14) comporte des moyens de retenue axiale (4a, 14a) destinés à coopérer avec des moyens de retenue axiale (6, 16) de l'élément d'assemblage à cône femelle (5, 15). La section droite délimitée par la face interne (4, 14) de la jupe tubulaire (3, 13) est constante, le profil circulaire de cette section droite étant de rayon variable, au moins une portion du profil circulaire de plus petit rayon formant les moyens de retenue axiale (4a, 14a).

## Description

La présente invention se rapporte à un élément d'assemblage étanche en matière plastique pour dispositif d'assemblage mâle-femelle de type Luer Lock, comprenant un cône mâle entouré d'une jupe tubulaire concentrique ménageant un espace annulaire autour du cône mâle pour recevoir un élément d'assemblage à cône femelle et dont la paroi interne comporte des moyens de retenue axiale destinés à coopérer avec des moyens de retenue axiale de l'élément d'assemblage à cône femelle. Elle se rapporte également à un dispositif d'assemblage mâle-femelle de type Luer Lock utilisant l'élément d'assemblage étanche en matière plastique.

On connaît bien dans le milieu de la perfusion et de la gestion des liquides biologiques (sang, etc.) les raccords Luer, basés sur l'emboîtement de deux cônes à 6% de conicité, mâle et femelle en matière plastique (selon la norme ISO 594/1). Ces raccords sont adaptés aux seringues et aux aiguilles hypodermiques, ainsi qu'à un certain nombre d'équipements médicaux.

L'assemblage du raccord se fait en exerçant une force axiale tout en appliquant une torsion, ce qui augmente le blocage des deux cônes.

On connaît bien également le raccord Luer Lock, comportant autour du cône mâle, une jupe dont la face interne de la paroi comporte un pas de vis, pour assurer le verrouillage du raccord et l'étanchéité selon la norme ISO 594/2.

La version Luer Lock de ce type d'assemblage a un coût de fabrication beaucoup plus élevé que le Luer simple, à cause de la complexité du moule d'injection, due à la présence du pas de vis. En effet, pour démouler un pas de vis, il faut que le moule comporte une partie mobile tournante, qui se dévisse de la pièce, avant l'éjection de cette dernière du moule.

Cette pièce mobile du moule est complexe, elle doit être motorisée (réalisation de deux révolutions en hélice). Le système est donc fragile et nécessite un entretien et des contrôles fréquents. Le temps de cycle est plus long. Tout cela explique le prix sensiblement plus élevé des pièces comportant un ou des raccords de ce type.

Un autre inconvénient des raccords Luer Lock provient du pas de vis, qui nécessite de tourner les deux éléments du raccord jusqu'à ce que l'on trouve l'entrée du pas de vis, avant de les tourner pour les visser l'un dans l'autre.

Le but de la présente invention est de remédier au moins en partie aux inconvénients sus-mentionnés.

A cet effet, la présente invention a tout d'abord pour objet un élément d'assemblage étanche en matière plastique pour dispositif d'assemblage mâle-femelle de type Luer Lock selon la revendication 1, cet élément étant utilisable avec un élément d'assemblage Luer Lock à cône femelle selon la norme ISO 594/2 et assure la compatibilité avec la plupart des raccords Luer Lock du marché.

Elle a également pour objet un dispositif d'assemblage mâle-femelle de type Luer Lock utilisant l'élément d'assemblage étanche en matière plastique de la revendication 1, selon la revendication 6.

L'avantage principal de cette invention résulte de la suppression du pas de vis ménagé sur la face interne de la jupe qui entoure le cône mâle de l'élément d'assemblage étanche, ce qui permet de supprimer la partie rotative du moule nécessaire pour démouler le pas de vis.

Un autre avantage vient du fait que les deux cônes mâle et femelle butent l'un contre l'autre avant d'effectuer une rotation de 90° environ à la suite de laquelle les moyens de retenue axiale de l'élément d'assemblage à cône femelle sont engagés dans les moyens de retenue axiale de l'élément d'assemblage à cône mâle du dispositif d'assemblage, sans avoir à chercher tout d'abord l'entrée du pas de vis et ne nécessitant ensuite qu'une rotation limitée de l'ordre d'un quart de tour, compatible avec l'amplitude de rotation du poignet. Il s'agit là d'une amélioration du point de vue ergonomique.

D'autres particularités et avantages apparaîtront au cours de la description qui suit. Celle-ci sera faite à l'aide des dessins annexés qui illustrent, schématiquement et à titre d'exemple, une forme d'exécution et des variantes de l'élément d'assemblage étanche et du dispositif d'assemblage utilisant cet élément d'assemblage, objets de la présente invention.
La figure 1 est une vue en perspective d'une forme d'exécution de l'élément d'assemblage étanche à cône mâle objet de l'invention;
la figure 2 est une vue en perspective d'un élément d'assemblage étanche à cône femelle selon la norme ISO 594/1;
la figure 3 est une vue en perspective avec coupe axiale du dispositif d'assemblage selon l'invention, obtenu par assemblage des éléments d'assemblage des figures 1 et 2;
la figure 4 est une vue en coupe selon IV-IV de la figure 3 avant l'engagement mutuel des moyens de retenue axiale;
la figure 5 est la même vue en coupe que celle de la figure 4, mais après la mise en prise mutuelle des moyens de retenue axiale;
la figure 6 est une vue en perspective d'une variante de la figure 1;
la figure 7 est une vue en perspective d'une variante de l'élément d'assemblage à cône femelle;
la figure 8 est une variante d'application utilisant le dispositif d'assemblage de type Luer-Lock selon l'invention;
la figure 9 est une vue en perspective d'une variante de la figure 1;
la figure 10 est une vue en perspective avec coupe de l'assemblage de la variante de la figure 7 avec l'élément de la figure 1.

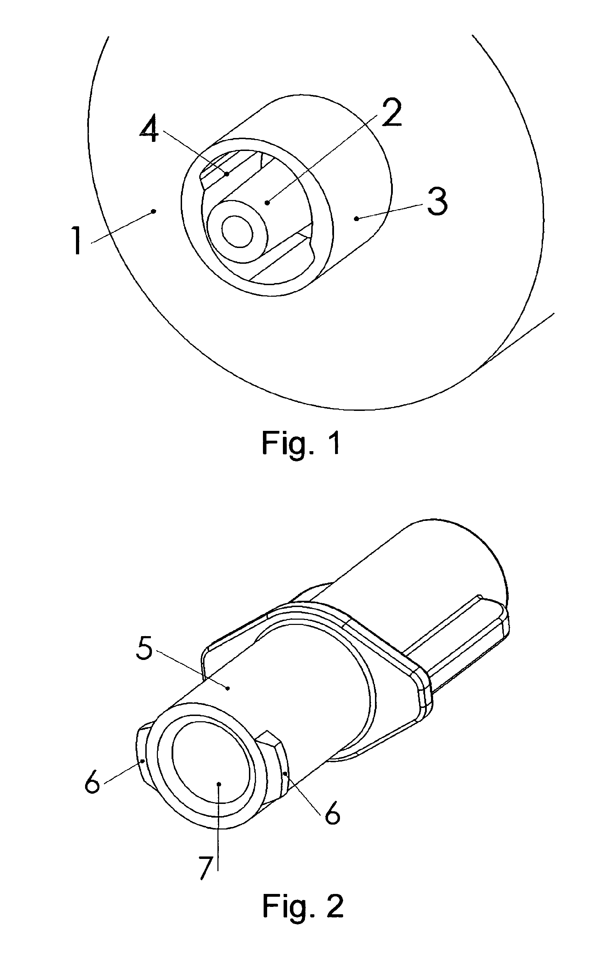

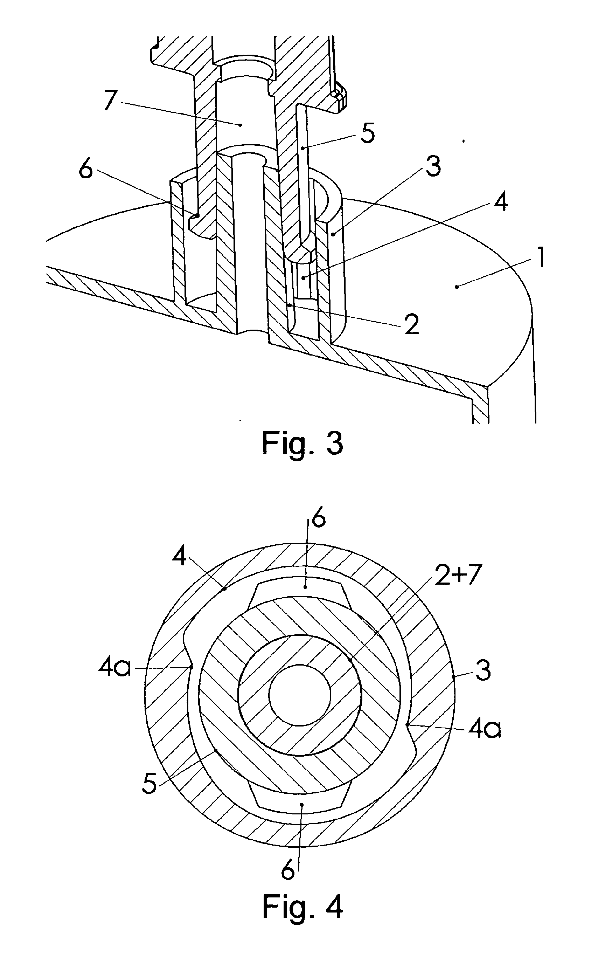

L'élément d'assemblage 1 illustré par la figure 1 comporte un cône mâle 2 avec un angle de 6%. Ce cône mâle 2 constitue un des organes de connexion d'un dispositif d'assemblage de type Luer Lock illustré par la figure 3. Une jupe tubulaire 3 entoure le cône mâle 2 et est coaxiale à ce cône 2. La face interne 4 de la jupe tubulaire 3 délimite un espace annulaire de section constante formant un cylindre curviligne. Le rayon de cette face interne 4 varie pour qu'au moins une portion du profil circulaire de plus petit rayon 4a forme les moyens de retenue axiale comme on l'expliquera ci-après. De préférence, le rayon du profil circulaire varie de manière périodique autour de l'axe du cône mâle 2. Avantageusement, le rayon du profil circulaire de la face interne 4 de la jupe 3 varie de manière symétrique autour de l'axe du cône mâle 2, comme illustré par la figure 1.

Cette face interne 4 présente avantageusement une forme de double spirale cylindrique symétrique.

L'élément d'assemblage à cône femelle 5 illustré par la figure 2 est un élément standard réalisé selon la norme ISO 594/1, qui comporte sur sa face externe deux saillies 6, constituant les moyens de retenue axiale et dont la face interne constitue le cône femelle 7.

Selon les dispositifs d'assemblage coniques standards, l'élément comprenant le cône mâle est généralement réalisé en polypropylène ou en polyéthylène (par exemple lorsqu'il fait partie intégrante d'une seringue), tandis que l'élément d'assemblage comportant le cône femelle est généralement réalisé en polycarbonate, PVC, PMMA, ABS ou autre matériau, sensiblement plus dur que le PP ou le PE utilisé pour l'élément d'assemblage 1 à cône mâle 2.

La figure 3 illustre les éléments d'assemblage 1 et 5 en position assemblée et la figure 4 est une vue en coupe selon IV-IV de la figure 3. Comme on peut le constater, dans cette position, les deux cônes, mâle 2 et femelle 7, sont en butée axiale, alors que les moyens de retenue axiale 4a et 6 ne sont pas engagés les uns avec les autres.

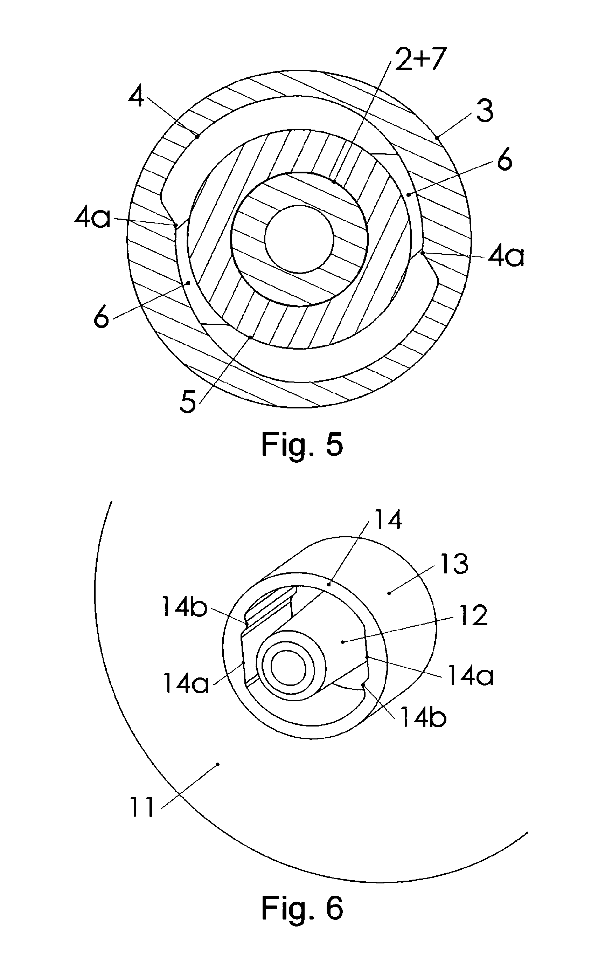

La figure 5 montre les élément d'assemblage 1 et 5 après que l'élément d'assemblage 5 à cône femelle 7 ait été tourné d'environ 90°. Etant donné que les rayons des parties de retenue axiale 4a de l'élément d'assemblage 1 sont inférieurs à ceux des éléments de retenue axiale 6 de l'élément d'assemblage 5 et que la jupe tubulaire 3 est en un matériau plastique relativement souple, cette jupe tubulaire 3 peut se déformer. Comme en plus, la matière plastique de la jupe 3 et donc des moyens de retenue axiale 4a est sensiblement plus tendre que celle des moyens de retenue axiale 6 de l'élément d'assemblage à cône femelle, comme on l'a mentionné précédemment, les moyens de retenue axiale 6 peuvent aussi déformer la matière des moyens de retenue axiale 4a pour y pénétrer.

La figure 6 illustre une variante de l'élément d'assemblage de la figure 1, dans laquelle la face interne 14 de la jupe 13 de cet élément d'assemblage 11 comporte deux parties symétriques 14a de plus petit diamètre formées par deux cordes parallèles d'un cercle formant la face interne 14, le cône mâle 12 restant inchangé.

Avantageusement, des éléments de blocage angulaire 14b sont destinés à limiter l'angle de rotation relative des deux éléments d'assemblage 11 et 5 ou 15 ci-après.

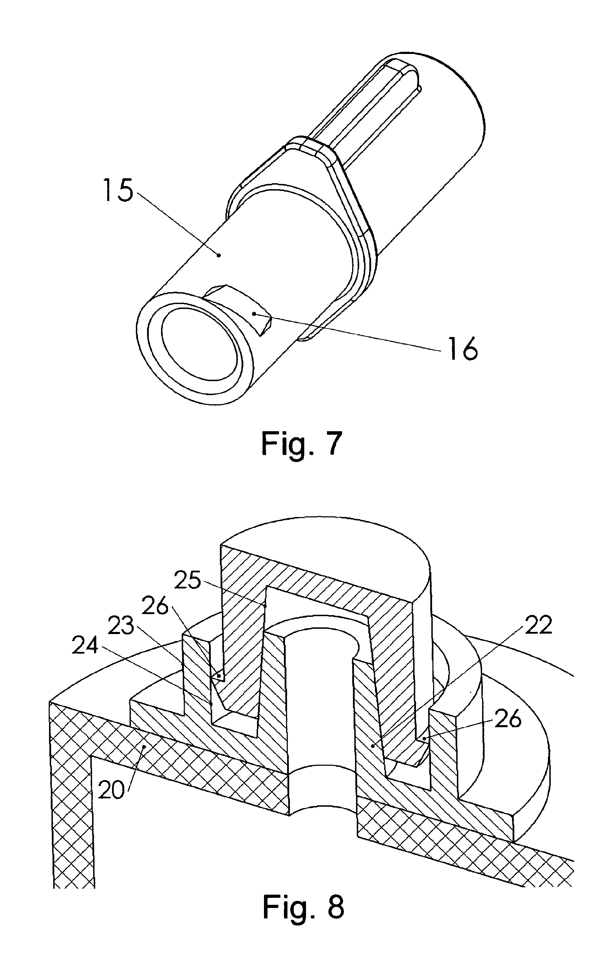

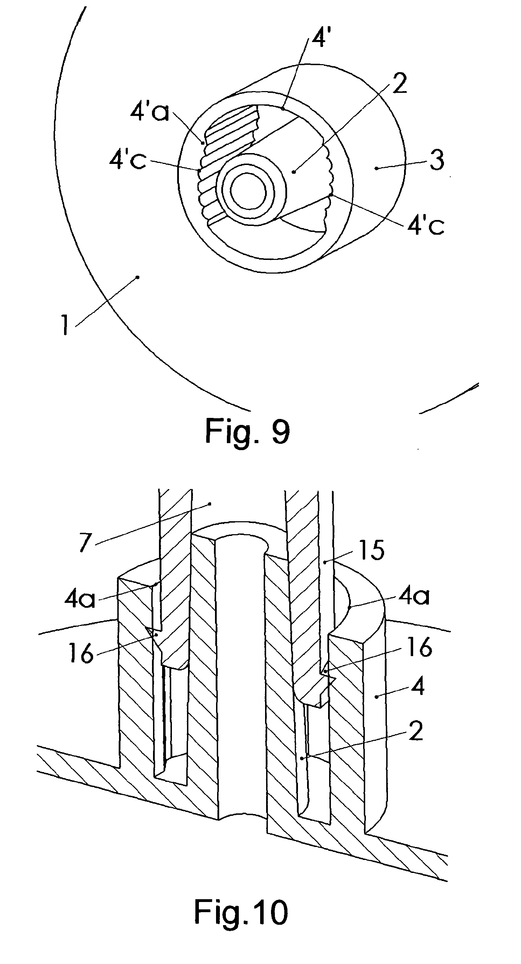

La figure 7 illustre une variante d'un élément d'assemblage 15 à cône femelle par rapport à celui de la figure 2. Ici, les saillies formant les moyens de retenue axiale sont formés par deux portions de pas de vis 16 de deux hélices décalées de 180°, dont seule l'une est visible sur la figure 7, qui permettent d'exercer une pression axiale sur les deux surfaces coniques mâle, respectivement femelle 2 et 7. De préférence, la section des deux portions 16 des pas de vis a la forme d'un triangle rectangle formant harpon , pour favoriser la pénétration dans les moyens de retenue 4a, 14a de la jupe 4, 14 de l'élément d'assemblage à cône mâle, comme illustré par la figure 10.

Grâce à la face interne 4 en forme de double spirale cylindrique symétrique et aux deux portions de pas de vis 16 de l'élément d'assemblage 15, on obtient un verrouillage axial amélioré.

La figure 8 montre que l'élément d'assemblage selon la présente invention ne se limite pas à un raccord Luer, mais peut aussi être utilisé dans d'autres applications. C'est ainsi que l'on peut utiliser l'élément d'assemblage à cône mâle 1 ou 11 décrit précédemment comme goulot 22 d'un récipient 20, entouré d'une jupe 23 dont la face inférieure 24 est de rayon variable, alors que l'élément d'assemblage à cône femelle 25 est un bouchon de fermeture étanche dont la face externe présente avantageusement deux moyens de retenue axiale 26 faisant saillie de cette face externe pour pénétrer dans les moyens de retenue axiale formés par les parties de plus petits rayons de la face interne 24 de la jupe 23. Dans une telle utilisation, la rainure annulaire ménagée entre le cône mâle 22 et la jupe 23 peut servir à recueillir les gouttes.

La variante illustrée par la figure 9 se rapporte au fait que la partie de la surface du cylindre curviligne 4' formant les moyens de retenue axiale 4'a est striée par des stries 4'c qui s'étendent parallèlement à la génératrice de la surface cylindrique curviligne 4'. Ces stries 4'c servent à faciliter la pénétration des éléments de retenue axiale 6 ou 16 de l'élément d'assemblage 5, 15 à cône femelle 7.

## Revendications

1. Elément d'assemblage conique étanche en matière plastique pour dispositif d'assemblage mâle-femelle de type Luer Lock, comprenant un cône mâle (2, 12) entouré d'une jupe tubulaire (3, 13) concentrique ménageant un espace annulaire autour du cône mâle pour recevoir un élément d'assemblage à cône femelle (5, 15) et dont la paroi interne (4, 14) comporte des moyens de retenue axiale (4a, 14a) destinés à coopérer avec des moyens de retenue axiale (6, 16) de l'élément d'assemblage à cône femelle (5, 15), **caractérisé en ce que** la section droite délimitée par la face interne (4, 14) de la jupe tubulaire (3, 13) est constante, le profil circulaire de cette section droite étant de rayon variable, au moins une portion du profil circulaire de plus petit rayon formant les moyens de retenue axiale (4a, 14a).

2. Elément selon la revendication 1, dans lequel le rayon du profil circulaire de la face interne (4, 14) de la jupe (3, 13) varie de manière périodique autour de l'axe du cône mâle.

3. Elément selon la revendication 2, dans lequel le rayon du profil circulaire de la face interne (4, 14) de la jupe (3, 13) varie de manière symétrique autour de l'axe du cône mâle.

4. Elément selon l'une des revendications précédentes, dans lequel la face interne (4, 14 de la jupe tubulaire comporte des moyens de blocage angulaire pour l'élément d'assemblage à cône femelle (5, 15).

5. Elément selon l'une des revendications précédentes, dans lequel au moins ladite portion du profil circulaire de plus petit rayon formant les moyens de retenue axiale comporte des stries parallèles à la génératrice dudit profil circulaire.

6. Dispositif d'assemblage mâle-femelle de type Luer Lock utilisant l'élément d'assemblage étanche en matière plastique selon la revendication 1, **caractérisé en ce que** la matière plastique de l'élément d'assemblage (1, 11) comprenant un cône mâle (2, 12) est de dureté sensiblement inférieure à celle de l'élément d'assemblage (5, 15) à cône femelle (7), pour permettre aux moyens de retenue axial (6, 16) de l'élément d'assemblage (5, 15) à cône femelle (7) de pénétrer dans au moins la portion du profil circulaire de plus petit diamètre (4a, 14a) du profil circulaire délimité par la face interne (4, 14) de la jupe tubulaire (3, 13) de l'élément d'assemblage (1, 11) à cône mâle (2, 12).

7. Dispositif selon la revendication 6, dans lequel les moyens de retenue axiale de l'élément d'assemblage à cône femelle comportent deux portions diamétralement opposées (16) de deux filets de deux pas de vis décalés de 180°.

8. Dispositif selon l'une des revendications 6 et 7, dans lequel les moyens de retenue axiale (16) de l'élément d'assemblage (15) à cône femelle sont des éléments saillant sur la face externe de cet élément d'assemblage ont une section droite triangulaire.
